Europäisches Patentamt

(19) European Patent Office

Office européen des brevets

(11) Publication number : **0 349 143 B1**

(12) **EUROPEAN PATENT SPECIFICATION**

(45) Date of publication of patent specification :
30.06.93 Bulletin 93/26

(51) Int. Cl.⁵ : **A61K 31/66**

(21) Application number : 89305930.3

(22) Date of filing : 12.06.89

(54) **Use of phytic acid or its salts for the prevention or treatment of hepatic diseases.**

(30) Priority : 01.07.88 JP 164717/88

(43) Date of publication of application :
03.01.90 Bulletin 90/01

(45) Publication of the grant of the patent :
30.06.93 Bulletin 93/26

(84) Designated Contracting States :
BE CH DE FR GB IT LI LU NL SE

(56) References cited :
PATENT ABSTRACTS OF JAPAN, vol. 8, no.
110 (C-224)[1547], 23rd May 1984; & JP-A-59 25
677 (KEI AI KAGAKU K.K.) 09-02-1984
JOURNAL OF VITAMINOLOGY, vol. 16, no. 1,
1970, pages 75-79; A. KOTAKI et al.: "Studies
on myoinositol. V. Effect of myoinositol on the
prevention of fatty liver induced by orotic
acid"
JOURNAL OF VITAMINOLOGY, vol. 17, 1971,
pages 112-116; I. NISHIGAKI et al.: "Studies
on myoinositol. VII. Removal of deposited fats
from dietary fatty liver"
J. NUTR., vol. 107, no. 10, October 1977, pages
1871-1883; L.E. BURTON et al.:
"Characterization of the lactation-dependent
fatty liver in myo-inositol deficient rats"
ANN. N.Y. ACAD. SCI., vol. 165, no. 2, 1969,
pages 710-725; H. YAGI et al.: "The effect of
massive doses of Myo-inositol on hepatic
phospholipid metabolism"

(73) Proprietor : SANWA KAGAKU KENKYUSHO
CO., LTD.
No. 35, Higashi-sotobori-cho
Higashi-ku Nagoya-shi Aichi-ken (JP)

(72) Inventor : Sawai, Kiichi
36-14 Ninomiya 1-chome
Funabashi-shi Chiba-ken (JP)
Inventor : Kurono, Masayasu
6-7 Sasaonishi 3-chome
Touincho Inabegun Mie-ken (JP)
Inventor : Asai, Hiromoto
1-6 Nakayamacho 5.chome Mizuho.ku
Nagoya-shi Aichi-ken (JP)
Inventor : Mitani, Takahiko
881-3 Ageki Hokuseicho-oaza
Inabe-gun Mie-ken (JP)
Inventor : Hayashi, Motohide
Kozyocho 261
Uto-shi Kumamoto-ken (JP)
Inventor : Nakano, Kazumasa
881-3 Ageki Hokuseicho-oaza
Inabe-gun Mie-ken (JP)
Inventor : Ninomiya, Naohisa
5-79 Motoyagoto Tenpaku-ku
Nagoya-shi Aichi-ken (JP)

(74) Representative : Diamond, Bryan Clive et al
Gee & Co., Chancery House, Chancery Lane
London WC2A 1QU (GB)

EP 0 349 143 B1

## Description

The present invention relates to the use of a pharmaceutical composition for treating and preventing hepatopathies or hepatic diseases and to such an edible composition for improving hepatic functions, which contain phytic acid or at least a salt thereof as a pharmaceutically effective component.

Hepatopathies, especially those produced by acquired causes such as drug-induced and viral hepatopathies are more frequently found in the elderly and so are a sort of geriatric disease. However, juveniles of relatively tender age tend to suffer from such diseases by reason of irregular ingestion, insanitariness, maternal infection, sexual infection and the like which often result in a serious condition. Because of the unavailability of any decisive remedies for their treatment, there is still an urgent need to provide effective remedies and preventives.

On the other hand, phytic acids widely appear in plants as calcium and magnesium salts, sometimes a potassium salt. For instance, rice bran contains as high as 9.5 to 14.5% of phytic acid and provides a starting material for commercial phytic acid and myoinositol derived therefrom.

Phytic acid and its salt have been used for many purposes in pharmaceutical applications, calcium phytate has been used as a calcium augmentative, rice bran itself and sodium phytate as a preventive for calcium calculus, and potassium phytate for the treatment of hyper-calcemia and hyper-calciurea or sarcoidosis patients. They have also been utilized in various other fields as fermentative aids for brewing sake and wine, metal removers making use of the chelating action of phytic acid, antioxidants in the presence of iron and calcium ions and anticorrosives for metals.

However, it has not been reported until now that phytic acid and its salts may be used as a preventive and remedy for hepatopathies and edible compositions for enhancing hepatic functions.

J. Vitaminol, 1970, 16(1), pp 75-9 discloses that inositol (myoinositol) fed to rats prevented accumulation of fat in the liver. The same journal, 1971, 17, pp 112-116 discloses that inositol when perfused into the fatty liver of a rat via the portal vein, accelerates improvement of fats from the liver thus curing the fat accumulation. Phytic acid has a molecule similar to that of inositol but differing by phosphorylation of the -OH substituents; inositol is expensively synthesised from natural phytic acid.

Surprisingly, the inventors have now discovered that when orally administered during nutrition experiments, phytic acid serves to reduce body smells, inter alia, foul breath, perspiratory smell and urinous smell. In particular, further research studies of the removal of alcoholic breath by phytic acid has revealed that phytic acid takes part in the production and decomposition of alcohols, inter alia, aldehydic substances that are in vivo metabolites and has the property of detoxicating them (Japanese Patent Application No. 63-116338), and that phytic acid serves to protect the liver.

In view of the aforesaid findings, the present invention provides a remedy and preventive for hepatopathies and an edible composition for enhancing hepatic functions, which contain phytic acid or at least a salt thereof as an effective component.

The remedies or preventives according to the present invention are administrable and suitable for both humans and animals. The compositions used herein, and specific examples thereof may be the same as disclosed in our EP-A-341810 wherein phytic acid is used as an antidote to poisoning by drugs or alcohol.

The present invention will now be described with reference to the accompanying illustrative drawings, in which:-

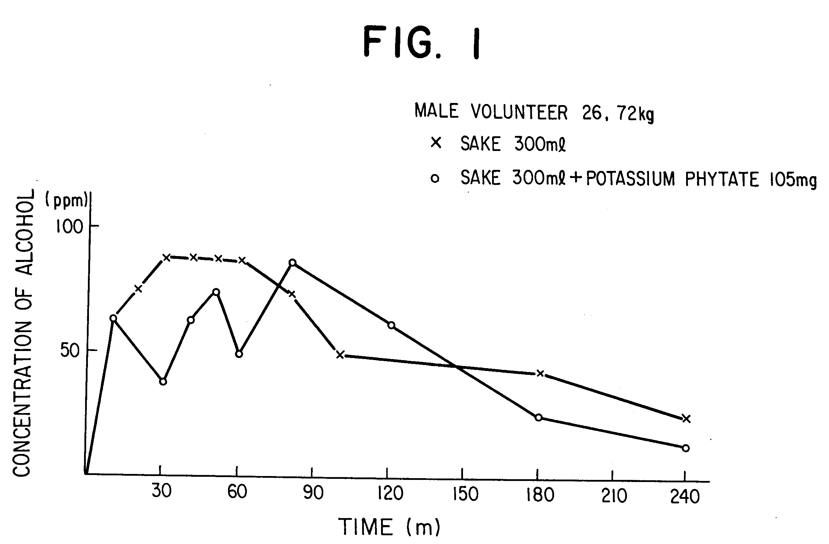

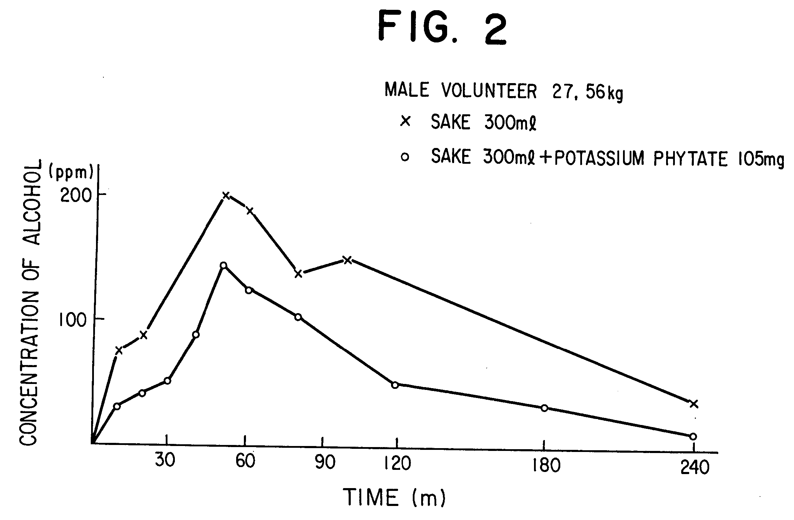

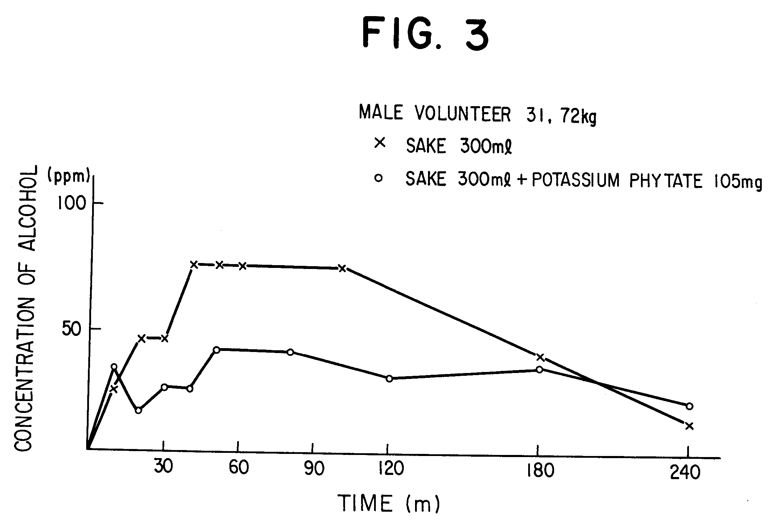

Figures 1 to 3 are graphs illustrating changes-with-time in the concentration of alcohol in breath, as measured in Example 1; Fig. 1 is a graph showing the results of testing with a male volunteer aged 26, Fig. 2 a graph showing the results of testing with a male volunteer aged 27, and Fig. 3 a graph showing the results of testing with a male volunteer aged 31.

In various preparations, phytates and their mixtures in a pH range of 6 to 8 may generally be selectively used depending upon the purposes of the pharmaceuticals and edible compositions because of their strong acidity.

The phytates usable in the present invention may include non-toxic metal salts as well as non-toxic salts with organic salts, basic amino acids and organic ester residues such as those represented by potassium phytate, sodium phytate, ammonium phytate, arginine phytate, ornithine phytate, lysine phytate, histidine phytate, monoethanolamine phytate, diethanolamine phytate, triethanolamine phytate and glucamine phytate.

The number of moles of various bases required to adjust one mole of phytic acid pH to 8 is shown in Table 1.

## Table 1

| Bases | pH: | 6.00 | 7.00 | 8.00 |
|---|---|---|---|---|
| NaOH | | 7.34 | 8.21 | 8.94 |
| KOH | | 7.34 | 8.23 | 8.94 |
| LiOH | | 7.41 | 8.38 | 9.30 |
| $NH_4OH$ | | 7.61 | 8.55 | 9.45 |
| $HOC_2HCH_2NH_2$ | | 7.72 | 8.68 | 9.52 |
| $(HOCH_2CH_2)_2NH$ | | 7.54 | 8.45 | 9.31 |
| $(HOCH_2CH_2)_3N$ | | 7.20 | 8.53 | 12.1 |
| N-Methylglucamine | | 7.62 | 8.49 | 9.25 |
| L-Arginine | | 7.79 | 8.67 | 9.60 |
| L-Lysine | | 8.01 | 8.98 | 10.0 |
| L-Histidine | | 11.3 | – | – |

Phytic acid and its salt are so tasteless and odorless that their oral administration is easily achieved. Thus, the compositions provided by the present invention may be administered by mixing with drinking water for humans and animals or sprinkling over or blending with dishes or feed in the form of powders or granules.

Furthermore, it is also possible to add various flavorings or other ingredients to various phytates or their mixtures to prepare elixirs, capsules, granules, powders, tablets, syrups, dry syrups, troches, candies, lemonade, drinkable solutions, garlic flavorings. It has been confirmed that phytic acid is very stable in such preparations.

A dosage of 1 to 100 mg/kg/day, calculated as phytic acid, of the compositions provided by the present invention may be suitable for humans, generally adults, although this depends upon the conditions of patients and the type of preparations.

To summarise, by use of the invention it is possible to easily obtain treatment and prevention of hepatopathies or enhancement of hepatic functions by the parenteral or oral administration of phytic acid and salts thereof.

Examples

The present invention will now be explained specifically with reference to the following examples.

Example 1

1: Pharmaceutical and Pharmacological Effect Tests

(1) Curative Effect Tests on Hepatopathy Models of Rats Carbon Tetrachloride

One (1) mg/kg of carbon tetrachloride was administered to a male rat weighing about 200 g twice a week over ten weeks to induce hepatopathy.

How much the hepatopathy was cured was determined in a conventional manner by the measurement of the eluting enzymes GOT (Glutamate-oxaloacetate transaminase) and GPT (Glutamine-pyruvate transaminase) due to hepatic cytoclasis to calculate the therapeutic index.

After the inducement of hepatopathy, 10 mg/kg of potassium phytate was continuously administered to a group of five rats by the intraperitoneal route for 14 days (physiological saline was administered to a control group) and blood was collected from the caudal veins for the measurement of GOT and GPT. As a consequence, a 45% or more increase in the therapeutic index was found. Vivisection also clearly indicated that the livers were ameliorated.

2: Effect upon the Amelioration of Drug Poisoning of Mice (Hepatic Function-Enhancing Tests)

Ten (10) mg/kg of potassium phytate was intraperitoneally administered to a group of six mice within 10 minutes of being anesthetized by the intraperitoneal injection of mg/kg of hexobarbital in order to compare arousal times. As a result, a 30% or more reduction in the arousal time was found. This appears to be due to the promoted decomposition and metabolism of the anesthetic in the liver since phytic acid has no hypersensitive or other substantial actions on the nerve system.

2. Clinical Tests

(1) Alcoholometry Testing (Metabolism Tests on the Enhancement of Hepatic functions)

After lunch, three male volunteers (age, weight:- 26, 72 kg; 27, 56 kg; 31, 72 kg) were alcoholized by the administration of 300 ml of saké (15% ethanol) to measure the concentration of alcohol in breath with the lapse of time.

Two days later, the same subjects were alcoholized by the administration of saké, immediately followed by the administration of 105 mg/10 ml of potassium phytate (pH 7.0), for the measurement-with-time of the concentration of alcohol in breath.

Plotted in Figures 1 to 3 are the results, from which it was found that the concentration of alcohol in breath was reduced by the administration of phytic acid.

3. Organoleptic Testing

After drink, the drinkable solution of Preparation Example 1 was dosed to three panelists fond of alcohol. On inquiry of their drinking habit one month later, all the panelists answered that there was a decrease in the amount of their drinking because of shortened intoxication time, limited drunken sickness, a feeling of fullness and increased aversion to drinking.

In order to estimate significantly the effect of the preparations upon the enhancement of hepatic functions, it is required to collect and study a number of cases. However, the present preparation was organoleptically concluded as satisfactory, since none of the panelists said otherwise.

Example 2

1. Preparation Examples

Composition a

Twenty-nine (29) g of sodium hydroxide and a suitable amount of refined water are added to 660 g of phytic acid (as an anhydride) to obtain a liquid adjusted to pH 6.

Composition b

Four hundred and twelve (412) g of potassium hydroxide and a suitable amount of refined water are added to 660 g of phytic acid (as an anhydride) to obtain a liquid adjusted to pH 6.

Composition c

One hundred and seventy-seven (177)g of lithium hydroxide and a suitable amount of refined water are added to 660 g of phytic acid (as an anhydride) to obtain a liquid adjusted to pH 6.

Composition d

Five hundred and eighty-one (581) g of ethanolamine and a suitable amount of refined water are added to 660 g of phytic acid (as an anhydride) to obtain a liquid adjusted to pH 8.

## Composition e

Nine hundred and seventy-nine (979) g of diethanolamine and a suitable amount of refined water are added to 660 g of phytic acid (as an anhydride) to obtain a liquid adjusted to pH 8.

## Composition f

One thousand eight hundred and five (1805) g of triethanolamine and a suitable amount of refined water are added to 660 g of phytic acid (as an anhydride) to obtain a liquid adjusted to pH 8.

## Composition g

One thousand six hundred and fifty-seven (1657) g of N-methylglucamine and a suitable amount of refined water are added to 660 g of phytic acid (as an anhydride) to obtain a liquid adjusted to pH 7.

## Composition h

One thousand five hundred and ten (1510) g of L-arginine and a suitable amount of refined water are added to 660 g of phytic acid (as an anhydride) to obtain a liquid adjusted to pH 7.

## Composition i

One thousand seven hundred and fifty-three (1753) g of L-histidine and a suitable amount of refined water are added to 660 g of phytic acid (as an anhydride) to obtain a liquid adjusted to pH 6.

## Composition j

One hundred and sixteen (116) g of sodium hydroxide, 478 g of potassium hydroxide, 6.08 g of potassium chloride (as a dihydrate), 157 g of disodium hydrogen phosphate (as an anhydride) and a suitable amount of refined water are added to 660 g of phytic acid (as an anhydride) to obtain a liquid adjusted to pH 9.

These compositions a to j may be powdered by crystallization or the addition of a vehicle.

These compositions a to j may also be formed into compositions in the form of liquids or powders, from which the preparations may be obtained.

## Example 3

The composition j obtained in Example 2 was formed into the compositions below, from which various preparations were obtained.

## Composition A for Preparations

Lactose is added to the composition j (containing 200 mg of phytic acid) to obtain a total of 1000 mg of a composition.

## Composition B for Preparations

Lactose is added to the composition j (containing 100 mg of phytic acid) to obtain a total of 1000 mg of a composition.

## Composition C for Preparations

Refined water is added to the composition j (containing 100 mg of phytic acid) to obtain a total of 1000 mg of a composition.

## Composition D

Light silicic anhydride is added to the composition j (containing 200 mg of phytic acid), followed by drying, which gives a total of 1000 mg of a composition.

Production Examples of Preparations

Production Example (Elixir)

Composition C               100 g (10 g calculated as phytic acid)
Compound orange extract    24 ml
Ethanol                    400 ml
Glycerine                 400 ml
Refined water       Total:    1000 ml

Predetermined amounts of the aforesaid components are uniformly mixed together to obtain a colorless and clear elixir preparation. A five-milliliter dosage of this elixir preparation contains 50 mg of phytic acid.

Production Example 2 (Capsule)

Composition A             200 mg (40 mg calculated as phytic acid)
Lactose                    20 mg
Corn starch            38 mg
Magnesium stearate      2 mg

Predetermined amounts of the aforesaid components are uniformly mixed together and packed in No. 2 capsules. One such capsule contains 40 mg of phytic acid.

Production Example 3 (Granule)

Composition A             600 mg (120 mg calculated as phytic acid)
Lactose                    140 mg
Corn starch            250 mg
Hydroxypropylcellulose    10 mg

Predetermined amounts of the aforesaid components are uniformly mixed together, and the mixture is then wet-granulated with water and ethanol into granules. One hundred and twenty (120) mg of phytic acid are contained in an one-gram dosage of such granules.

Production Example 4 (Powder)

The composition A is divided and heat-sealed in aluminium to obtain wrappers each of 1.5g of powder.

Production Example 5 (Tablet)

Composition A             100 mg (20 mg calculated as phytic acid)
Corn starch            19 mg
Crystalline cellulose     30 mg
Magnesium stearate      1 mg

Predetermined amounts of the aforesaid components are uniformly mixed together, and the mixture is then compressed into tablets each of 7 mm in diameter and 150 mg in weight. One such tablet contains 20 mg of phytic acid.

Production Example 6 (Syrup)

Composition C             50 g (5 g calculated as phytic acid)
White sugar            300 g
D-sorbitol (70%)        250 g
Methyl p-oxybenzoate    0.3 g
Propyl p-oxybenzoate     0.15 g
Sodium citrate          10 g
Perfume                   1.5 g
Refined water       Total:   1000 ml

Predetermined amounts of the aforesaid components are dissolved and mixed together into a colorless and clear syrup. One hundred (100) mg of phytic acid is contained in a twenty-milliliter dosage of this syrup.

Production Example 7 (Dry syrup)

| Composition B | 100 mg (10 mg calculated as phytic acid) |
|---|---|
| Sodium citrate | 2.4 mg |
| Citric anhydride | 2.2 mg |
| Tragacanth powders | 2.7 g |
| White sugar | suitable amount |
| Hydroxypropylcellulose | 3.0 mg |
| Perfume | slight amount |
| Perfume | slight amount |

Predetermined amounts of the aforesaid components are uniformly mixed together, and are then wet-granulated with water and ethanol into a dry syrup. An one (1)-gram dosage of this syrup contains 10 mg of phytic acid.

Production Example 8 (Troche)

| Composition A | 100 mg (20 mg calculated as phytic acid) |
|---|---|
| White sugar | 870 mg |
| Lactose | 20 mg |
| Magnesium stearate | 10 mg |

Of the aforesaid components the composition A and white sugar are uniformly mixed together in the respective amounts of 100 g and 870 g, and are then wet-granulated with water and ethanol, followed by drying at a temperature of lower than 35°C. Added to the dried product are 20 g of lactose and 10 g of magnesium stearate to obtain troches each of 15 mm in diameter and 1 g in weight. One such troche contains 20 mg of phytic acid.

Production Example 9 (Candy)

| Composition B | 100 mg (10 mg calcuated as phytic acid) |
|---|---|
| White sugar | 2400 mg |
| Starch syrup | 1500 mg |
| Perfume | slight amount |

Of the aforesaid components, 240 g of white sugar and 150 g of starch syrup are mixed with 100 g of refined water. After melting by heating, the mixture is sieved for the removal of foreign matters. The resulting liquid is concentrated under pressure with the application of heat for dehydration to prepare a starch syrup dough having a moisture content of 2 to 3 % at 130 to 150°C. Added to this dough are 10 g of the composition B and a slight amount of perfume, and the product is molded to obtain candies each of 4 g in weight. Each candy contains 10 mg of phytic acid.

Production Example 10 (Magnesium Citrate Oral Solution)

| Composition C | 3 g (300 mg calculated as phytic acid) |
|---|---|
| Syrup | 2.5 ml |
| Refined water | Total: 30 ml |

Predetermined amounts of the aforesaid components are uniformly mixed together into "lemonade". A thirty (30)-milliliter dosage of such lemonade contains 300 mg of phytic acid.

Production Example 11 (Granules)

| Composition D | 500 mg (100 mg calculated as phytic acid) |
|---|---|
| Garlic powders | 750 mg |
| Lactose | suitable amount |

Predetermined amounts of the aforesaid components are uniformly mixed together, and are then wet-granulated with water and ethanol into granules. One hundred (100) mg of phytic acid is contained in an 1.5-gram dosage of such granules.

Production Example 12 (Drinkable Solution)

| Composition C | 1 g (100 mg calculated as phytic acid) |
| Mel | 0.5 g |
| White sugar | 2.0 g |
| Citric acid | suitable amount |
| Sodium citrate | suitable amount |
| Peppermint | slight amount |
| Refined | water suitable amount |

Predetermined amounts of the aforesaid components were uniformly mixed together into a colorless and clear internal liquid preparation. A thirty (30)-milliliter dosage of this liquid preparation contains 100 mg of phytic acid.

Production Example 13 (Garlic Flavoring)

| Composition D | 0.285 g (0.1 g calculated as phytic acid) |
| Avicel (Cellulose microcrystalline) | 0.18 g |
| Garlic powders | 0.75 g |
| Light silicic anhydride | 0.256 g |
| Corn starch | suitable amounts |

Predetermined amounts of the aforesaid components are granulated by a conventional method.

Stability Testing

The preparations according to Production Examples 1 to 12 were subjected to stability testing to measure the amount of residual phytic acid. The results are set forth in Table 2.

## Table 2

Amounts of Residual Phytic Acid in the Stability Testing of the Preparations According to the Production Examples (% with respect to the specified contents)

| Samples | Storage Vessels | At the beginning of Storage | After 3 weeks at 60°C |
|---|---|---|---|
| P.Ex.1A* | Glass Bottle | 100.5 | 101.2 |
| P.Ex.2B* | PTP | 101.4 | 99.4 |
| P.Ex.3C* | Aluminium Wrapper | 100.1 | 100.0 |
| P.Ex.4D* | " | 100.9 | 102.1 |
| P.Ex.5E* | PTP | 99.2 | 99.8 |
| P.Ex.6F* | Glass Bottle | 102.1 | 100.3 |
| P.Ex.7G* | Aluminium Wrapper | 100.6 | 100.1 |
| P.Ex.8H* | Aluminium SP | 99.7 | 100.5 |
| P.Ex.9I* | Aluminium Bag | 99.9 | 99.2 |
| P.Ex.10J* | Glass Bottle | 102.1 | 100.9 |
| P.Ex.11K* | Aluminium Wrapper | 100.3 | 100.1 |
| P.Ex.12L* | Glass Bottle | 100.1 | 99.8 |

A*: Elixir, B*: Capsule, C*: Granule, D*: Powder, E*: Tablet,
F*: Syrup, G*: Dry Syrup, H*: Troche, I*: Candy, J*: Limonada,
K*: Granule, L*: Drinkable Solution.

## Claims

1. Use of phytic acid and/or a salt thereof for the manufacture of a medicament for the prevention or treatment of hepatic diseases or improvement of hepatic function.

2. Use, as claimed in Claim 1, wherein the hepatic diseases are drug-induced or viral hepatic diseases.

3. Use, as claimed in Claim 1 or 2, wherein the medicament is a composition in a form suitable for oral administration.

4. Use, as claimed in any preceding claim, wherein the salt of phytic acid is a non-toxic metal salt, or a non-toxic salt with an organic base, a basic amino acid or an organic ester residue.

5. Use, as claimed in Claim 4, wherein the salt of phytic acid is selected from potassium phytate, sodium phytate, ammonium phytate, arginine phytate, ornithine phytate, lysine phytate, histidine phytate, monoethanolamine phytate, diethanolamine phytate, triethanolamine phytate and glucamine phytate.

6. Use, as claimed in any preceding claim, wherein the phytic acid and/or a salt thereof are orally administered daily.

**Patentansprüche**

1. Verwendung von Phytinsäure und/oder eines Salzes davon zum Herstellen eines Medikamentes zum Verhindern oder zum Behandeln von Leberkrankheiten, oder zum Verbessern der Leberfunktion.

2. Verwendung nach Anspruch 1, wobei die Leberkrankheiten durch Drogen oder Viren verursachte Leberkrankheiten sind.

3. Verwendung nach Anspruch 1 oder 2, wobei das Medikament ein Präparat in einer Form ist, die sich für die orale Einnahme eignet.

4. Verwendung nach einem der vorangehenden Ansprüche, wobei das Salz der Phytinsäure ein nicht-toxisches Metallsalz, oder ein nicht-toxisches Salz einer organischen Base, einer basischen Aminosäure, oder eines organischen Ester-Restes ist.

5. Verwendung nach Anspruch 4, wobei das Salz der Phytinsäure Kalium-Pythat, Natrium-Pythat, Ammonium-Phytat, Arginin-Phythat, Ornithin-Phythat, Monoethanolamin-Phythat, Diethanolamin-Phythat, Triethanolamin-Phythat oder Glucamin-Phythat ist.

6. Verwendung nach einem der vorangehenden Ansprüche, wobei die Phytinsäure und/oder ein Salz davon, täglich, oral zu verabreichen ist.

**Revendications**

1. Utilisation d'acide phytique et/ou d'un sel de celui-ci pour la fabrication d'un médicament pour traiter ou prévenir les maladies hépatiques ou pour améliorer la fonction hépatique.

2. Utilisation selon la revendication 1, où les maladies hépatiques sont induites par un médicament, ou ce sont des maladies hépatiques virales.

3. Utilisation selon la revendication 1 ou 2, où le médicament est une composition sous une forme adaptée à l'administration par voie orale.

4. Utilisation selon l'une quelconque des revendications précédentes, où le sel de l'acide phytique est un sel de métal non toxique ou un sel organique non toxique, un sel d'acide aminé basique ou il s'agit d'un ester organique.

5. Utilisation selon la revendication 4, où le sel de l'acide phytique est choisi parmi le phytate de potassium, le phytate de sodium, le phytate d'ammonium, le phytate d'arginine, le phytate d'ornithine, le phytate de lysine, le phytate d'histidine, le phytate de monoéthanolamine, le phytate de diéthanolamine, le phytate de triéthanolamine et le phytate de glucamine.

6. Utilisation selon l'une quelconque des revendications précédentes, où l'acide phytique et/ou un sel de celui-ci est administré par voie orale sur une base journalière.

# FIG. 1

MALE VOLUNTEER 26, 72kg

× SAKE 300mℓ

o SAKE 300mℓ + POTASSIUM PHYTATE 105mg

EP 0 349 143 B1

# FIG. 2

MALE VOLUNTEER 27, 56kg

× SAKE 300mℓ

○ SAKE 300mℓ+POTASSIUM PHYTATE 105mg

CONCENTRATION OF ALCOHOL (ppm)

TIME (m)

EP 0 349 143 B1

# FIG. 3

MALE VOLUNTEER 31, 72kg

× SAKE 300mℓ

o SAKE 300mℓ + POTASSIUM PHYTATE 105mg

EP 0 349 143 B1